# EUROPEAN PATENT SPECIFICATION

(11) **EP 1 960 030 B1**
(45) Date of publication and mention of the grant of the patent: **09.06.2010**
(21) Application number: 06838726.5
(22) Date of filing: 01.12.2006
(51) Int. Cl.: A61M 25/09

(54) **GUIDEWIRE WITH PERFUSION CAPABILITY**
FÜHRUNGSDRAHT MIT PERFUSIONSFÄHIGKEIT
FIL-GUIDE À CAPACITÉ DE PERFUSION

(30) Priority: 02.12.2005 US 292538
(43) Date of publication of application: 27.08.2008
(73) Proprietor: Boston Scientific Limited, an Irish company, Barbados, West Indies (BB)
(72) Inventor: EIDENSCHINK, Tracee, Wayzata, Minnesota 55391 (US)
(74) Representative: Peterreins, Frank
(86) International application number: PCT/US2006/045913
(87) International publication number: WO 2007/064832

(56) References cited:
- WO-A-01/68176
- WO-A-94/10919
- WO-A2-99/62584
- US-A- 3 841 308
- US-A- 6 123 698

## Description

### TECHNICAL FIELD

This invention relates to medical devices, and more particularly to guidewires that are used, for example, with catheters and that have the capability of delivering and injecting fluid within a body vessel.

### BACKGROUND

Guidewires are widely used in conjunction with therapeutic devices, such as catheters, which are threaded over the guidewire to gain access to an area within the body requiring diagnosis or treatment. Typically, a guidewire has a significantly smaller outer diameter than a therapeutic device and can therefore be inserted into the body to access remote areas more easily. When the guidewire is positioned properly within the body, the therapeutic device may be passed over and guided along the guidewire to a desired location. The therapeutic device is then utilized to diagnose and/or treat the area at the location. For example, a balloon catheter may be used as the therapeutic device to perform a medical procedure, such as a percutaneous transluminal coronary angioplasty (PTCA).

Evidence suggests that early reperfusion of blood into the heart, after removing a blockage to blood flow, dramatically reduces damage to the ischemic tissue region and to the myocardium. However, the reestablishment of blood flow may cause a reperfusion injury to occur. It is possible to reduce reperfusion injury by cooling the affected region prior to reperfusion. One method of cooling myocardial tissue is to place an ice pack over the patient's heart. Another method involves puncturing the pericardium and providing cooled fluid to a reservoir inserted into the pericardial space near the targeted myocardial tissue. Cooling of the myocardial tissue may also be accomplished by perfusing the target tissue with cooled solutions, such as by supplying cooled fluid to the target tissue through a catheter placed in the patient's blood vessel.

Guidewires have been produced that can be used to deliver a fluid. These guidewires allow exit of the fluid through the end of the guidewire, or through a porous perfusion zone, for example US 6 123 698 A, WO 01/68176 A, US 3 841 308 A, WO 99/62584 A2, WO 94/10919 A.

### SUMMARY

In accordance with the present invention a guidewire is provided as defined in claim 1. Further advantages are achieved by the embodiments indicated by the dependent claims.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1 is a side view of an embodiment of a perfusing guidewire located within a balloon catheter.
FIG. 2 is a side view of a distal portion of the perfusing guidewire of FIG. 1 in a retracted state.
FIG. 3 is a side view of a distal portion of the perfusing guidewire of FIG. 1 in an expanded state.
FIG. 4 is a longitudinal cross-section of a distal portion of the perfusing guidewire of FIG. 1 in a retracted state, as shown in FIG. 2.
FIG. 5 is a cross-section of the guidewire of FIG. 2 and FIG. 4, along the line 5-5 shown in FIG. 2 and in FIG. 4.
FIG. 6 is a cross-section of the guidewire of FIG. 2 and FIG. 4, along the line 6-6 shown in FIG. 2 and in FIG. 4.
FIG. 7 is a longitudinal cross-section of a distal portion of the perfusing guidewire of FIG. 1 in an expanded state, as shown in FIG. 3.
FIG. 8 is a side view of a distal portion of an alternative embodiment of a perfusing guidewire in a retracted state.
FIG. 9 is a cross-section of the guidewire of FIG. 8, along the line 9-9 shown in FIG. 8.
FIG. 10 is a side view of a distal portion of an example of a perfusing guidewire in a retracted state.
FIG. 11 is a side view of a distal portion of the perfusing guidewire of FIG. 10 in an expanded state.
FIG. 12 is a diagram of a side view of a proximal end of a perfusing guidewire in one embodiment, positioned in a coronary artery, with a balloon catheter deployed over the perfusing guidewire, and illustrates a method of treating a target tissue region near the heart.
FIG. 13 is a diagram of a side view of the proximal end of a perfusing guidewire in one embodiment connected to a control system, with the control system shown in block diagram.

Like reference symbols in the various drawings indicate like elements.

### DETAILED DESCRIPTION

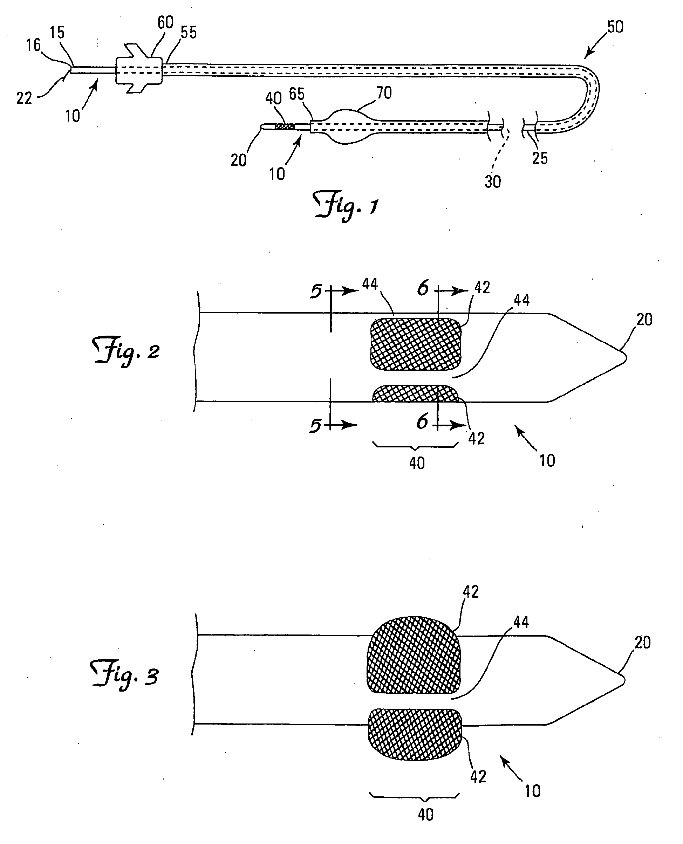

FIG. 1 shows a balloon catheter 50 and one embodiment of a guidewire with perfusion capability 10. The perfusing guidewire 10 may be inserted and used as a standard, conventional guidewire. For example, it may be used to guide a distal end 65 of the catheter 50 to a desired treatment location within a body.

The perfusing guidewire 10 includes an elongate member 25 and has a perfusion lumen 30 (not shown in FIG. 1) extending longitudinally therethrough from the proximal end 15 of the guidewire 10 to a perfusing section 40 located near a distal end 20 of the guidewire 10. Fluid may be introduced into this lumen at a fluid entry port 16 at the proximal end 15 of the guidewire 10. Fluid travels through the guidewire lumen 30 to the perfusing section 40, where it exits the guidewire 10. The guidewire 10 may be left in the body during a balloon procedure so that perfusion may be done before, during, and/or after the time that the balloon is blocking blood flow through a body vessel during the procedure.

The perfusing guidewire 10 is constructed so that the perfusing section 40 is expandable when perfusing a fluid. This is done to reduce the pressure of the fluid at the point of departure from the lumen to prevent or minimize damage to body tissue that otherwise might occur. In one embodiment (shown later in more detail) for example, the perfusing section 40 may be expanded by pulling on a wire 22 running from the proximal end 15 of the perfusing guidewire 10 to near the distal end 20 of the guidewire 10.

As described later in more detail, the perfusing guidewire may be inserted into a body, such as via an artery, and advanced to a target location. A therapeutic device, such as a balloon catheter, may then be advanced over the guidewire to the target location. Fluid may be perfused through the perfusion guidewire before, during, and after a medical procedure conducted using the therapeutic device.

In more detail, the perfusing guidewire 10 of FIG. 1 is shown located within a balloon catheter 50. The balloon catheter 50 includes a proximal end 55, a distal end 65, and an expandable balloon 70 located near the distal end 65 of the catheter 50. An adapter 60 is also shown attached to the proximal end 55. The outer diameter of the perfusing guidewire 10 is sized to allow the passage of the distal end 65 of the balloon catheter 50 over the proximal end 15 of the perfusing guidewire 10, and allow the catheter 50 to be advanced over the member 25 of the perfusing guidewire 10 to reach a desired position. Generally, when positioned correctly and ready for treatment, the distal end 20 of the perfusing guidewire 10 will remain distal of the distal end 65 of the balloon catheter 50. At that same time, the proximal end 15 of the perfusing guidewire 10 will generally remain proximal of the proximal end 55 of the balloon catheter 50 as well as proximal of the adapter 60.

The perfusing guidewire 10 includes a perfusion section 40 located near the distal end 20 of the perfusing guidewire 10. The perfusion lumen 30 extends longitudinally from a fluid entry port 16 located at the proximal end 15 of the perfusing guidewire 10 to the perfusion section 40. A wire 22 is shown in FIG. 1, and runs from the proximal end 15 to near the distal end 20 of the perfusing guidewire 10.

FIGS. 2 and 3 show more detail of a distal portion of the perfusing guidewire 10 shown in FIG. 1. FIG. 2 shows the perfusion section 40 of the guidewire 10 in a retracted state, while FIG. 3 shows the perfusion section 40 in an expanded state. The perfusion section 40 includes perfusion areas 42 through which fluid from the perfusion lumen exits, and solid areas 44. Solid areas 144 may also be referred to as a plurality of longitudinally extending ribs. The perfusion section 40 shown in FIGS. 2 and 3 has three perfusion areas 42, of which only two are shown. There are also three solid areas 44, of which only two are shown. The other perfusion area and solid area are on the back side of the perfusion section 40 shown in FIGS. 2 and 3, and are thus hidden from view. The perfusion areas 42 in FIGS. 2 and 3 are covered with a mesh material.

In the expanded state, the area of the perfusion areas 42 increases compared to the retracted state. Also, in an expanded state, the diameter of the midpoint of the perfusion section 40 has an increased diameter compared to the midpoint of the perfusion section 40 in a retracted state. The mesh material covering the perfusion areas 42 expands to allow the perfusion areas 42 to increase in area. This increases the area from which the fluid will leave the perfusion lumen, passing through the perfusion areas 42. This reduces the pressure of the fluid leaving the perfusing guidewire 10, and decreases the risk and severity of tissue injury. In addition, the mesh material also serves a tissue protection function, partially deflecting the fluid exiting from the perfusion lumen through the perfusion areas 42.

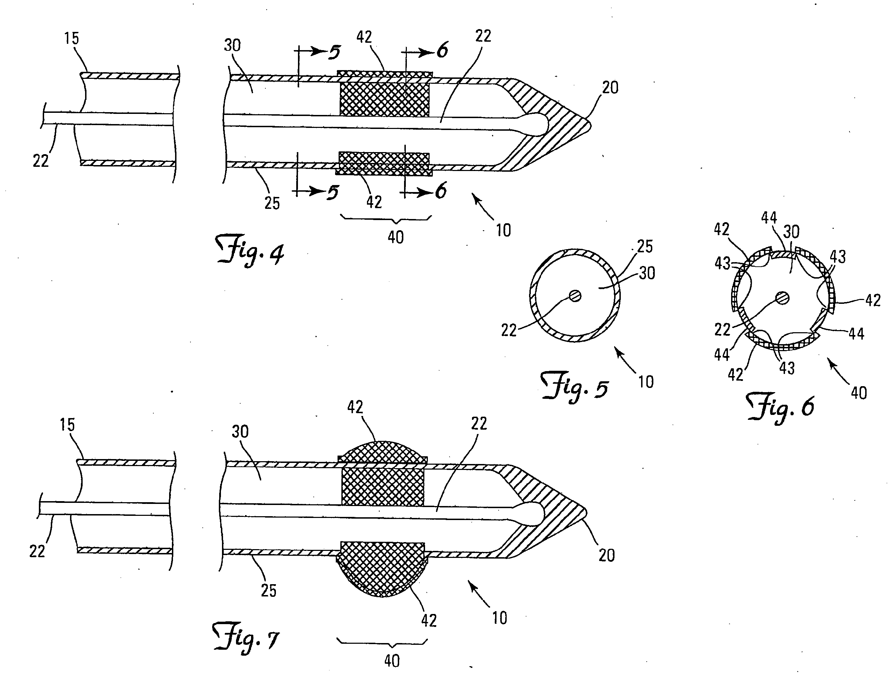

FIGS. 4 and 7 show a longitudinal cross-section of the distal end of the perfusing guidewire 10 shown in FIG. 1. These figures correspond to the side view of the perfusing guidewire 10 as shown in FIGS. 2 and 3. The member 25 of the perfusing guidewire 10 includes a perfusion lumen 30, formed by the walls of the member 25. A wire 22 runs from near the proximal end 15 to near the distal end 20 of the perfusing guidewire 10. The wire 22 is embedded near the distal end 20 of the perfusing guidewire.

FIG. 4 shows the perfusion section 40 in a retracted state. The perfusion section 40 is compressed in length by pulling on the wire 22, which is embedded near the distal end 20 of the perfusing guidewire 10. As the perfusion section 40 compresses lengthwise, the solid areas 44 are forced to bow outwards, increasing the diameter of the midpoint of the perfusion section 40. This also causes the perfusion areas 42 to move outward also, expanding the mesh material. This opens the perfusion areas 42, allowing fluid to more readily exit from the perfusion lumen 30 through the perfusion areas 42 and out of the guidewire. FIG. 7 shows the perfusion section 40 in an expanded state.

FIG. 5 shows a cross-section of the perfusing guidewire 10 taken along lines 5-5 of FIG. 2 and FIG. 4. The perfusion lumen 30 is formed by the walls of the guidewire member 25. The wire 22 is also shown, and is located within the perfusion lumen. In other embodiments, the pull wire running from near the proximal end to near the distal end of the perfusing guidewire may be located within the outer wall of the guidewire member.

FIG. 6 shows a cross section of the perfusion guidewire 10 in the perfusion section 40, taken along lines 6-6 of FIG 2 and FIG. 4. The perfusion section 40 is shown retracted in FIG. 6. The perfusion lumen 30 is formed at this point by the perfusion areas 42 and solid areas 44. The wire 22 is also shown located in the perfusion lumen. In FIG. 6, the perfusion areas 42 are bonded to the outside of the solid areas 44 at bonding points 43.

Introducing a fluid into the body may create a risk of perfusion damage caused by supplying a fluid to an area in a body vessel. This risk may be caused by the fluid pressure and flow. This risk is especially great when using a guidewire. In order to achieve an effective degree of cooling using a guidewire lumen, high fluid pressure is usually necessary in order to supply an effective amount of fluid through the guidewire to the target location. However, unrestricted exit of a fluid from a small exit area, such as a distal end of a guidewire, may cause jetting of fluid. Thus, fluid exiting at high pressure through a small exit area includes a high degree of risk of a large amount of vessel damage.

However, the increased area of the perfusion areas 42 of the perfusion section 40 serves to reduce the local pressure forces so that fluid exiting the perfusion areas 42 has a decreased risk of damaging vessel tissue. The use of an expanded area for fluid passage into the body allows a fluid to be provided at higher infusion rates without producing the same level of jetting effects in the body. The flow rate of the fluid (Q) is approximately equal to the velocity of the fluid (V) multiplied by the area of the exit location (A), Q = V * A. Therefore, increasing the area through which the fluid passes enables the same fluid flow rate to be achieved while using a lower velocity. Alternatively, a higher flow rate may be achieved while using the same velocity.

In general, the perfusion areas 42 may be a single area, or may be multiple areas. The area(s) may be entirely open, or may be covered by a mesh, filter, or other partially transmissible material of some type. If there are multiple areas 42, the areas may be all the same size and shape, or may be different sizes and shapes. The areas 42 may be rectangular, elliptical, circular, or other shape. When covered with a mesh, filter, or other transmissible material, the perfusion areas 42 allow minimal passage of fluid from the perfusion lumen 22 through the perfusion areas 42 when the perfusing guidewire 10 is in a retracted state, as shown in FIG. 2.

The diameter of the perfusion lumen 30 will be large enough to pass different types of fluid through the perfusion lumen. The fluid to be passed may be blood, saline, or other fluid. The fluid may be cooled. The fluid may include an amount of a drug. Examples of the types of drugs that may be used include anticoagulants, anti-clotting agents, and anti-inflammatories.

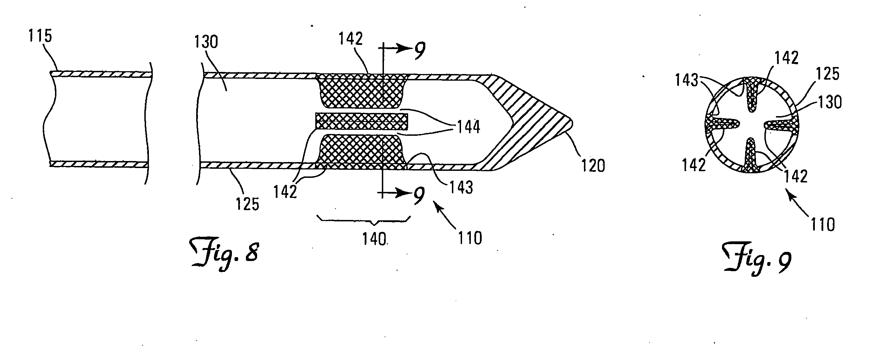

FIGS. 8 and 9 show another embodiment of a perfusing guidewire. These figures show an area near the distal end of a perfusing guidewire 110, and include an elongate member 125 and a perfusion area 140 near the distal end 120 of the perfusing guidewire. The elongate member 125 includes a perfusion lumen 130, formed by the walls of the elongate member 125. The perfusion section 140 includes perfusion areas 142 and solid areas 144. The perfusion areas 142 allow minimal passage of fluid from the perfusion lumen 130 through the perfusion areas 142 when the perfusion section 140 is in a retracted state, as shown in FIG. 8.

FIG. 9 shows a cross-section of the perfusing guidewire 110 taken along lines 9-9 of FIG. 8. The perfusion areas 142 and solid areas 144 are also shown. The perfusion areas 142 are bonded to the edges of solid areas 144 at bonding points 143. The perfusion areas 142 extend nearly to the center of the perfusion lumen 130 in an unexpanded state.

The perfusion areas 142 in perfusion section 140 are pushed out by the pressure of a cooled fluid. As the perfusion areas 42 expand, they open, allowing fluid to readily exit from the perfusion lumen 130 through the perfusion areas 142 and out of the guidewire. After perfusion is complete, the perfusion areas 142 warm to body temperature, causing the perfusion areas to retract to nearly the initial state, allowing the guidewire 110 to be removed from the body.

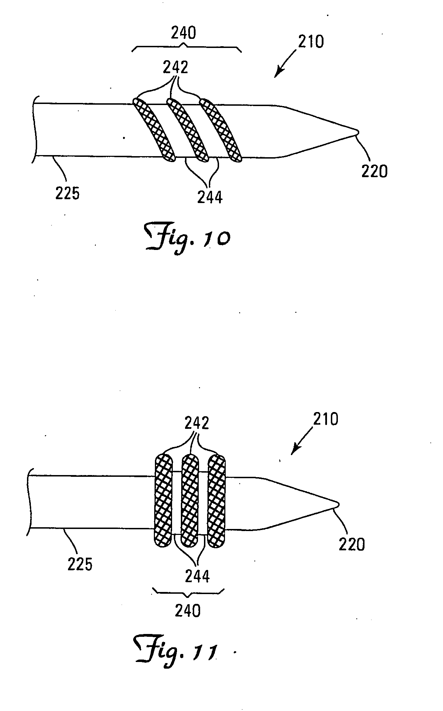

FIGS. 10 and 11 show an example of a perfusing guidewire. These figures show an area near the distal end of the perfusing guidewire 210, and include an elongate member 225 and a perfusion area 240 near the distal end 220 of the perfusing guidewire. The perfusion section 240 includes perfusion areas 242 and solid areas 244. The perfusion areas 242 allow minimal passage of fluid from the perfusion lumen (not shown) through the perfusion areas 242 when the perfusion section 240 is in a retracted state, as shown in FIG. 10. When the perfusion section 240 is in a retracted state, the perfusion areas 242 are in a twisted configuration.

When a rotational (or twisting) force is applied to the perfusing guidewire 210 in the proper manner, the rotational force is transmitted through the elongate member 225 and acts to twist perfusion section 240. This rotational force results in an untwisting and deployment of the perfusion section 240. As shown in FIG. 11, when the perfusion section 240 is deployed, the cross section diameter increases as the perfusion areas 242 untwist. This opens the perfusion areas 242 and expands the area for passage of fluid from the perfusion lumen through the perfusion areas 242 and out of the perfusing guidewire 210.

In some examples, a proper rotational force action might be a counterclockwise twisting of the perfusing guidewire to untwist the perfusion section. In such a case, the perfusing guidewire might be guided to a proper location in a body utilizing only clockwise twisting and turning of the perfusing guidewire. Then, perfusion would commence after a counterclockwise twisting deployed the perfusion section 240, as shown in FIG. 11. After perfusion in complete, the perfusion section 240 could be returned to the original, retracted, configuration by clockwise twisting.

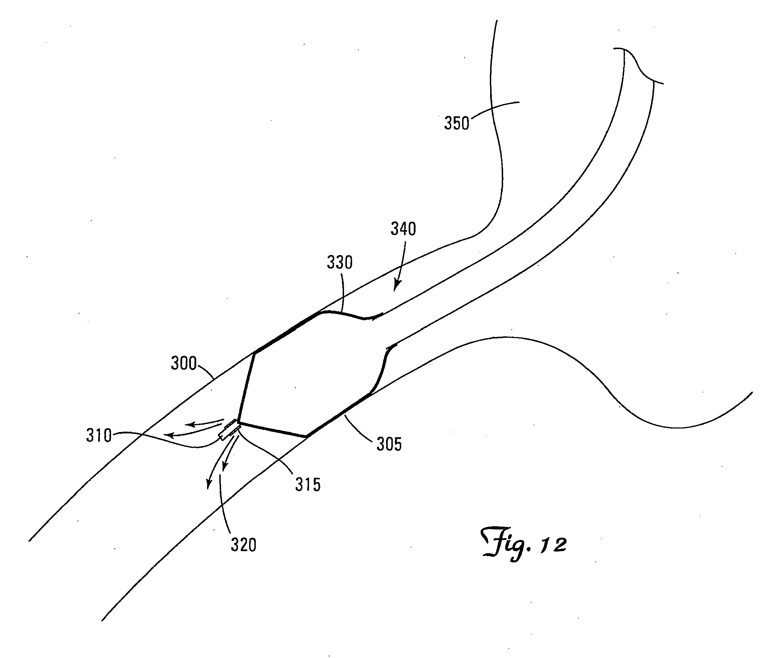

FIG. 12 shows an application of using a perfusing guidewire simultaneously and in conjunction with other components during a procedure. In the retracted state, the perfusing guidewire 310 may be used in a similar fashion as other guidewires. Also illustrated in FIG. 12 is a method of treating a target tissue region located in a body vessel. A perfusing guidewire 310 may be inserted into a body vessel, such as a femoral artery, that provides access to a patient's aorta 350. The guidewire may be advanced through the femoral artery into and through the patient's aorta 350, and into a desired vessel, such as a coronary artery 300.

After the perfusing guidewire is in place, a catheter or other therapeutic devices may then be advanced over the perfusing guidewire 310 for various treatments. For example, a balloon catheter 340 may be advanced over the perfusing guidewire to a target location 305 in a body vessel. In FIG. 12, a distal portion of a balloon catheter 340 is shown inside a coronary artery 300 near a patient's heart.

Once positioned in the coronary artery 300, fluid may be passed through the perfusing guidewire 310 and out of perfusion areas located in perfusion section 315. This introduces a fluid 320 distally of a target region 305 of the coronary artery 300. The fluid 320 may be cooled in order to cool tissue past the target tissue region 305 of the coronary artery 300. Fluid may be perfused before, during, or after a therapeutic procedure.

At the same time as fluid is being perfused, a balloon portion 330 of the balloon catheter 340 may be inflated to treat the target tissue region 305. One such treatment includes using the balloon 330 to treat and repair a lesion in the coronary artery 300 that has reduced or blocked the flow of blood through the vessel. After treatment, the deflation of the balloon 330 allows the resumption of blood flow through the coronary artery. The flow of fluid 320 may be stopped after treatment, or continued for a period of time. The catheter may also be removed while cooling continues.

Alternatively, the small cross section of the perfusing guidewire 310 in a retracted state allows the perfusing guidewire to be retracted through a catheter or other instrument, if desired.

The perfusing guidewire may be controlled manually, or may be used to perform treatments automatically with the aid of an external control system.

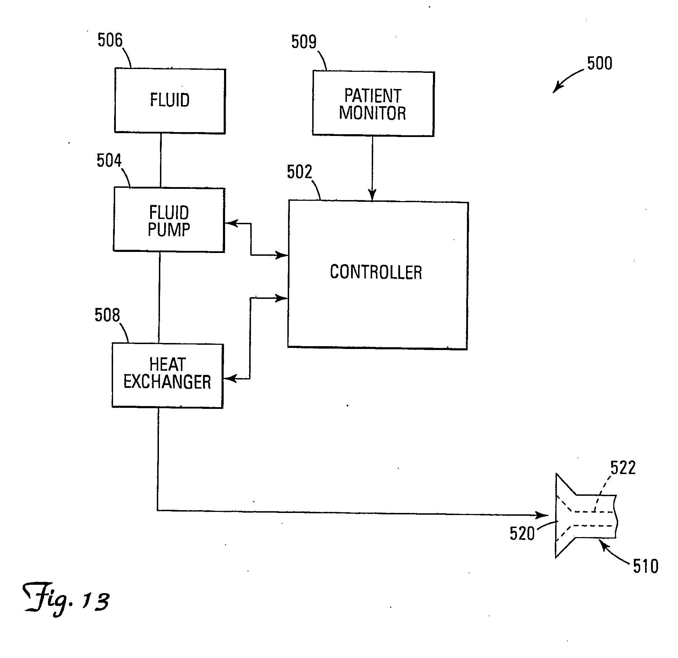

FIG. 13 shows a control system 500 along with a fluid entry port 520 located at the proximal end of a perfusing guidewire 510. In this example, control system 500 includes a controller 502, a fluid pump 504, a heat exchanger 508, and a patient monitor 509. The controller 502 controls the operation of the fluid pump 504 and the heat exchanger 508, which together dictate the temperature and rate of fluid provided to a target tissue region via the perfusing guidewire 510.

In FIG. 13, the controller 502 receives input from the other devices in the control system 500 and uses that input, in addition to patient data input manually, or via a link to another system, to coordinate the providing of fluid to a tissue region. For example, the fluid pump 504 provides the controller 502 with the rate at which the fluid 506 is infused through the perfusing guidewire 510 to the tissue region. The patient monitor 509 provides the patient's physiologic data to the controller 502, such as the patient's heart rate, heart rhythm, blood pressure, blood oxygen level, etc. From this information, the controller 502 can provide an alarm or alert the doctor that the patient is experiencing complications, or may adjust the treatment procedure automatically. Optionally, a temperature monitor may be used to provide information about a temperature of the target region, or the fluid pump 504 or heat exchanger 508 might provide temperature information about the fluid.

The controller 502 may also receive information about the procedure to be performed, including the specific perfusing guidewire to be used, the cooling technique to be used, the vessel which will be treated, the type of fluid being applied to the target tissue region, a schedule profile of fluid pressure and perfusion rates, the total length of the procedure, the target temperature range for the fluid, the total volume of fluid to be used, and other information. The control data may also include procedural constraints, such as the maximum infusion rate of the fluid, the pressure at which the fluid should be perfused, a maximum and minimum temperature for the target tissue region, as well as other possible constraints. These are only examples of some of the control data that may be provided to the controller 502 to control a treatment procedure.

After receiving the patient data, inputs from other devices, any manually entered information, and any control data, the controller 502 processes the information in accordance with the control data and provides output to the various components of the control system 500. For example, it provides output to the fluid pump 504, and the heat exchanger 508 to control the infusion rate and temperature of fluid 506. During the procedure, the controller 502 continually monitors the progress of the procedure and adjusts the outputs in accordance with the procedure being performed. The controller 502 may be a digital, analog, or other type of controller.

The fluid 506 used for perfusion may be any biocompatible fluid. The fluid selected will be based upon the purpose of the procedure. The fluid 506 may also contain additives that may be changed during the procedure. These additives may be added to the fluid 506 using a pump not shown in FIG. 13. The fluid 506 may be perfused through the perfusing guidewire using a pump 504. The fluid 506 may be provided to a fluid entry port 520 where the fluid passes into a perfusion lumen 522 in the perfusion guidewire 510. For example, a positive displacement pump may be used to provide the necessary pressure to push the fluid through the perfusion lumen to the distal end of the perfusion guidewire (not shown in FIG. 13). In some implementations, the pump 504 may be replaced by a manual system including a raised bag of fluid, where gravity, or a pressure cuff may be used to control the rate of the fluid 506. The fluid pump 504 may include a perfusion monitor to monitor the pressure and flow rate of the fluid through the perfusion lumen. It may also include a temperature sensor to monitor the temperature of the fluid.

If the fluid 506 is to be warmed or cooled, a conventional heat exchanger may be used. In one implementation, the heat exchanger 508 is controlled by the controller 502 by processing the information received from temperature sensors and manual input. Based on the information provided to the controller 502, the heat exchanger 508 may be used to cool or warm the fluid 506 provided to the target tissue region.

In various embodiments, the perfusing guidewire may be sized with different diameters. Common cross-sectional diameters of guidewires range from fourteen thousandths of an inch (.014) (0,356 mm) up to 35 thousandths of an inch (.035) (0,889 mm). The cross-sectional diameter of the perfusing guidewire may have the same range of diameters. The perfusion section of the guidewire may have the same or only slightly larger diameter in a retracted state than the member of the perfusing guidewire. For example, the outer diameter of the elongated member distal portion in a retracted state (or perfusion section) may have a diameter that is less than about 0.03 inches (0,762 mm) more than the diameter of the elongated member at the longitudinal midpoint between the proximal end and the distal end of the elongated member (or midpoint of the perfusing guidewire). Variously, the diameter of the perfusion section in a retracted state may be less than about 0.02 inches (0,508 mm) more than the diameter of the midpoint of the perfusing guidewire, or may be less than about 0.01 inches (0,254 mm) more than the diameter of the midpoint of the perfusing guidewire.

The midpoint of the perfusion section of the perfusing guidewire when expanded may have a diameter that is at least about 1.25 times the diameter of the midpoint of the perfusing guidewire. In other embodiments, the midpoint of the perfusion section of the perfusing guidewire when expanded may have a diameter that is at least about 1.5 times the diameter of the member of the perfusing guidewire, or may have a diameter that is at least about 2.0 times the diameter of the member of the perfusing guidewire.

The diameter of the perfusion section will increase during perfusion. In various embodiments, the outer diameter of the midpoint of the perfusion section in an expanded state may have a diameter that is at least about 1.25 times, at least about 1.5 times, or at least about 2.0 times the diameter of the same point when the perfusion section is in a retracted state.

In general, the sum of the area of the perfusion areas when expanded is greater than the cross-sectional area of the member of the perfusing guidewire. In some embodiments, the sum of the area of the perfusion areas when expanded will be at least about 1.25 times the cross-sectional area of the member of the perfusion guidewire. In other embodiments, the sum of the area of the perfusion areas when expanded will be at least about 1.5 times the cross-sectional area of the member of the perfusing guidewire, or at least about 2.0 times the cross-sectional area of the member of the perfusing guidewire.

In other embodiments, a mesh may completely encase the perfusion section of the perfusion guidewire and be bonded along the outside of the solid areas. In other embodiments, a mesh may be bonded to the inside of the solid areas. In various embodiments, the perfusion areas may be covered by mesh, filter, or other transmissible material.

In an alternate embodiment, a wire may be connected to a length of tube near the perfusion section of the perfusion guidewire. When the wire is pulled, the tube is pulled proximally uncovering the perfusion section. As the fluid reaches the perfusion section, the fluid causes the perfusion section to expand in diameter. This allows fluid to pass through the perfusion areas. When perfusion is complete, the tube may be advanced, ending perfusion, and causing a return to the original retracted configuration. In various embodiments, the length of tube may be inside the perfusion section, or on the outside of the guidewire covering the perfusion section.

In other embodiments, the solid areas in the perfusion section may include bending rods. These bending rods may be made of a different material than the solid areas. For example, the solid areas may be made of the same material as the outer walls of the member. Bending rods, made of a different material, may be inserted into the solid areas. Bending rods may be made of a material that bends outward under the pressure of pulling the wire, expanding the diameter of the perfusion section. This may assist in opening the perfusion section. In addition, the bending rods would generally exert a pressure to unbend. Therefore, when the wire is released, the bending rods act on the perfusion section to assist in returning the perfusion section to the retracted state.

In other embodiments, the perfusion section may include shape memory material. The solid areas and/or the perfusion areas may be partly or completely formed of shape memory material, or the solid areas may include bending rods made of a shape memory material. The shape memory material will change shape in reaction to some stimulus, such as, for example, a change in temperature. The response causes the shape memory material to change shape, compressing the length of the perfusion section and increase the diameter of the midpoint of the perfusion section. This expands the perfusion areas for the passage of fluid from the perfusion lumen through the perfusion areas and out of the perfusing guidewire. When the stimulus ceases, then the shape memory material may return to the original configuration. Examples of suitable shape memory materials include shape memory metal compositions, such as nitinol, and shape memory polymers, such as polycyclooctane. A system incorporating a shape memory material may also include a wire as a backup system, or may not include a wire at all. In addition, an electroactive polymer may be used. An electroactive polymer, such as polypyrrole nafion, is a polymer which responds to electrical stimulation by undergoing a significant shape or size displacement.

For example, in one embodiment, the shape memory material may respond to a decrease in temperature. As a cooled fluid is pumped into the perfusing guidewire and advances along the perfusion lumen it eventually reached the perfusion section. When the cooled fluid reaches the perfusion section, the temperature change causes the shape memory material to respond and change shape. The continued passage of the cooled fluid maintains the shape memory material in the responsive state, keeping the perfusion section in an expanded configuration. When the desired amount of fluid has been perfused, the passage of fluid will cease and the perfusion section will increase in temperature. As the temperature increases, the shape memory material in the perfusion section will return to the original configuration, returning the perfusion section to a retracted configuration. For example, a shape memory polymer may be manufactured that has an extended shape at body temperature (37°C) and a contracted/bent shape at a lower temperature (for example, 32°C). Bending rods may be formed from this shape memory polymer and embedded in the solid areas of the perfusion section. Then, when the cooled fluid cools the perfusion section, the bending rods will contract and bend, expanding the diameter of the perfusion section, allowing fluid to exit the perfusion lumen through the perfusion areas. The continued passage of cooled fluid will keep the shape memory polymer in the contract/bent configuration. After perfusion is complete, the passage of cooled fluid will cease, and the fluid in the perfusion section will increase in temperature. Once the temperature increases sufficiently, the shape memory polymer will return to the original, extended configuration, restoring the perfusion section to the contracted configuration.

In another embodiment, the perfusion section is constructed so that the perfusion section will expand in response to the application of internal pressure. As a fluid is pumped into the perfusing guidewire and advances along the perfusion lumen it eventually reached the perfusion section, increasing the pressure. The perfusion section expands in response to the increased pressure. This expansion leads to an expansion of the perfusion areas and passage of fluid through the perfusion areas from the perfusion lumen. When the desired amount of fluid has perfused, the passage of fluid will cease, causing a pressure decrease, with a resulting return of the perfusion section to the original, retracted, configuration.

In another embodiment, the perfusion section is constructed such that it is light activated to return to the initial configuration. For example, the perfusion section could expand due to a temperature or pressure change, allowing perfusion. Then, when perfusion is complete, the section could be exposed to light, causing a retraction of the perfusion areas. The light might be provided by a fiber optic running through the perfusion lumen, or through a wall of the device to the perfusion sections. Alternatively, light might be provided via an optical sheath over the entire wire allowing light to reach the perfusion section. Alternatively, light might be provided via an internal coating on the inside of the walls forming the perfusion lumen.

In another embodiment, the perfusion section is constructed such that it heat or electrically activated to return to the initial configuration. For example, the perfusion section could expand due to a temperature or pressure change, allowing perfusion. Then, when perfusion is complete, the section could be exposed to heat or electric current, causing a retraction. The temperature might be a return to body temperature, as discussed earlier, or it might be an elevated temperature. For example, warm saline could be perfused to increase the temperature of the perfusion section, causing the perfusion areas to retract. Alternatively, a current might be applied via a wire running through the perfusion section or through the wall of the device to the perfusion section. The current might cause a retraction of the perfusion areas, or might cause the perfusion section to increase in temperature, causing the retraction. Alternatively, a current might be run through the guidewire it self, causing an electrical based retraction, or leading to an increase in temperature of the perfusion section, causing a temperature based retraction.

The perfusion guidewire may be used with other therapeutic devices other than a balloon catheter. For example, the perfusion guidewire may be used with filters, intravascular ultrasound, stents, etc. The perfusion guidewire may also be used independently in addition to being used in conjunction with another device. For example, the perfusion guidewire may be used to perfuse an amount of fluid in a target location in a small vessel.

A number of embodiments of the invention have been described Nevertheless, it will be understood that various modifications may be made without departing from scope of the invention. Accordingly, other embodiments are within the scope of the following claims.

## Claims

1. A guidewire (10, 110, 310, 510) for use with a therapeutic device, the guidewire (10, 110, 310, 510) comprising:
an elongate member (25, 125) shaped for insertion into a body vessel such that a therapeutic device can be guided over the elongate member (25, 125) and into the body vessel, the elongate member (25, 125) having a perfusion lumen (30, 130) extending therethrough from a fluid entry port (16, 520) to exit openings included in a distal portion of the elongate member (25, 125),
**characterised in that**
the distal portion of the elongated member (25, 125) including a plurality of longitudinally extending ribs (44, 144) and expandable mesh material included between each of the ribs (44, 144), each of the ribs (44, 144) extending for a distance from near the distal tip of the elongate member (25, 125), wherein the mesh material covers the exit openings, wherein said distal portion construction has a retracted state that facilitates introduction of the elongate member (25, 125) into and through the body vessel and an expanded state where the area of the exit openings in the distal portion of the elongate member (25, 125) is increased.

2. The guidewire (10,110,210,310,510) of claim 1, wherein the guidewire (10,110,210,310,510) is used with a balloon catheter (50,340), and is sized such that a balloon catheter (50,340) can be guided over the elongate member (25,125,225).

3. The guidewire (10,110,210,310,510) of claims 1 or 2, wherein the longitudinally extending ribs extend generally straight in the retracted state and generally bow outward in the expanded state.

4. The guidewire (10,110,210,310,510) of one of the preceding claims, wherein the longitudinally extending ribs comprise bending rods which extend generally straight in the retracted state and generally bow outward in the expanded state.

5. The guidewire (10,110,210,310,510) of one of the preceding claims, further comprising:
a wire attached to the elongate member distal tip, wherein pulling the elongate structure proximally causes a distal end of the elongate member distal portion to be pulled nearer to a proximal end of the elongate member distal portion, which causes the longitudinally extending ribs to bow outwardly.

6. The guidewire (10,110,210,310,510) of claim 5, wherein the elongate structure extends proximally from an attachment point at the elongate member distal tip, within the elongate member perfusion lumen (30,130), and out of the perfusion lumen (30,130) near the elongate member proximal end.

7. The guidewire (10,110,210,310,510) of one of the preceding claims, wherein the longitudinally extending ribs are formed from a composition comprising a shape memory material.

8. The guidewire (10,110,210,310,510) of one of the preceding claims, wherein the expandable mesh material is formed from a composition comprising a shape memory material.

9. The guidewire (10,110,210,310,510) of claim 7 or 8, wherein the shape memory material comprises nitinol or polycyclooctane.

10. The guidewire (10,110,210,310,510) of one of the preceding claims, wherein the outer diameter of the midpoint of a perfusion section located at the elongated member distal portion in an expanded state has a diameter that is at least about twice the diameter of the same point when the elongated member distal portion is in a retracted state.

11. The guidewire (10,110,210,310,510) of one of the preceding claims, wherein a distal portion of the elongate member (25,125,225) is expandable upon the application of a cooled fluid (320,506) to the distal portion of the elongate member (25,125,225) from the perfusion lumen (30,130).

12. A system for performing a balloon catheter procedure, the system comprising:
a guidewire (10,110,210,310,510) according to one of the preceding claims;
an expandable balloon catheter (50,340); and
a control system (500) that controls the perfusion of fluid (320,506) through the guidewire perfusion lumen and out of the exit openings in the distal portion of the elongate member (25,125,225).

13. The system of claim 12, further comprising a control system (500) that controls the inflation and deflation of the expandable balloon (70).

14. The system of claims 12 or 13, wherein the control system (500) controls an amount of cooled fluid (320,506) that the guidewire (10,110,210,310,510) provides to a target region, so as to cool and maintain the target region within a target temperature range below normal body temperature for an extended period of time.

## Patentansprüche

1. Führungsdraht (10, 110, 310, 510) zur Verwendung mit einer therapeutischen Vorrichtung, der Führungsdraht (10, 110, 310, 510) umfassend:
ein längliches Element (25, 125) geformt zum Einsetzen in ein Körpergefäß, sodass eine therapeutische Vorrichtung über das längliche Element (25, 125) und in das Körpergefäß geführt werden kann, wobei das längliche Element (25, 125) ein Perfusionslumen (30, 130) aufweist, das sich **dadurch** von einem Flüssigkeitseintrittsanschluss (16, 520) zu Ausgangsöffnungen erstreckt, die in einem distalen Teil des länglichen Elementes (25, 125) beinhaltet sind,
**dadurch gekennzeichnet, dass**
der distale Teil des länglichen Elementes (25, 125) eine Mehrzahl von sich longitudinal erstreckenden Rippen (44, 144) und aufweitbares Maschenmaterial beinhaltet zwischen jeder der Rippen (44, 144), wobei sich jede der Rippen (44, 144) über eine Distanz von nahe der distalen Spitze des länglichen Elements (25, 125) erstreckt, wobei das Maschenmaterial die Ausgangsöffnungen bedeckt, wobei die Konstruktion des distalen Teils einen eingefahrenen Zustand aufweist, der Einführen des länglichen Elementes (25, 125) in und durch das Körpergefäß ermöglicht, und einen aufgeweiteten Zustand, in dem die Fläche der Ausgangsöffnungen im distalen Teil des länglichen Elementes (25, 125) erhöht ist.

2. Führungsdraht (10, 110, 210, 310, 510) nach Anspruch 1, wobei der Führungsdraht (10, 110, 210, 310, 510) mit einem Ballonkatheter (50, 340) verwendet wird und ausgelegt ist, sodass ein Ballonkatheter (50, 340) über das längliche Element (25, 125, 225) geführt werden kann.

3. Führungsdraht (10, 110, 210, 310, 510) nach Ansprüchen 1 oder 2, wobei die sich longitudinal erstreckenden Rippen sich im eingefahrenen Zustand im Allgemeinen gerade erstrecken und im aufgeweiteten Zustand im Allgemeinen nach außen biegen.

4. Führungsdraht (10, 110, 210, 310, 510) nach einem der vorhergehenden Ansprüche, wobei die sich longitudinal erstreckenden Rippen Biegestäbe umfassen, die sich im eingefahrenen Zustand im Allgemeinen gerade erstrecken und im aufgeweiteten Zustand im Allgemeinen nach außen biegen.

5. Führungsdraht (10, 110, 210, 310, 510) nach einem der vorhergehenden Ansprüche, weiter umfassend:
einen Draht, befestigt an der distalen Spitze des länglichen Elementes, wobei proximales Ziehen der länglichen Struktur bewirkt, dass ein distales Ende des distalen Teils des länglichen Elementes näher an ein proximales Ende des distalen Teils des länglichen Elementes gezogen wird, was bewirkt, dass sich die sich longitudinal erstreckenden Rippen nach außen biegen.

6. Führungsdraht (10, 110, 210, 310, 510) nach Anspruch 5, wobei die längliche Struktur sich proximal von einem Befestigungspunkt an der distalen Spitze des länglichen Elementes erstreckt, innerhalb des Perfusionslumens (30, 130) des länglichen Elementes, und aus dem Perfusionslumen (30, 130) hinaus nahe dem proximalen Ende des länglichen Elements.

7. Führungsdraht (10, 110, 210, 310, 510) nach einem der vorhergehenden Ansprüche, wobei die sich longitudinal erstreckenden Rippen aus einer Zusammensetzung gebildet sind, die ein Formgedächtnismaterial umfasst.

8. Führungsdraht (10, 110, 210, 310, 510) nach einem der vorhergehenden Ansprüche, wobei das aufweitbare Maschenmaterial aus einer Zusammensetzung gebildet ist, die ein Formgedächtnismaterial umfasst.

9. Führungsdraht (10, 110, 210, 310, 510) nach Anspruch 7 oder 8, wobei das Formgedächtnismaterial Nitinol oder Polyzyklooktan umfasst.

10. Führungsdraht (10, 110, 210, 310, 510) nach einem der vorhergehenden Ansprüche, wobei der äußere Durchmesser des Mittelpunktes eines Perfusionsabschnittes, der sich an dem distalen Teil des länglichen Elementes in einem aufgeweiteten Zustand befindet, einen Durchmesser aufweist, der mindestens ungefähr das Doppelte des Durchmessers des selben Punktes ist, wenn der distale Teil des länglichen Elementes im eingefahrenen Zustand ist.

11. Führungsdraht (10, 110, 210, 310, 510) nach einem der vorhergehenden Ansprüche, wobei ein distaler Teil des länglichen Elementes (25, 125, 225) bei der Anwendung einer gekühlten Flüssigkeit (320, 506) auf den distalen Teil des länglichen Elementes (25, 125, 225) aus dem Perfusionslumen (30, 130) aufweitbar ist.

12. System zur Durchführung eines Ballonkatheterverfahrens, das System umfassend:
einen Führungsdraht (10, 110, 210, 310, 510) gemäß einem der vorhergehenden Ansprüche;
einen aufweitbaren Ballonkatheter (50, 340); und
ein Steuerungssystem (500), das die Perfusion von Flüssigkeit (320, 506) durch das Führungsdraht-Perfusionslumen und aus den Ausgangsöffnungen in dem distalen Teil des länglichen Elementes (25, 125, 225) steuert.

13. System nach Anspruch 12, weiter umfassend ein Steuerungssystem (500), das das Aufblähen und Entleeren des aufweitbaren Ballons (70) steuert.

14. System nach Ansprüchen 12 oder 13, wobei das Steuerungssystem (500) eine Menge von gekühlter Flüssigkeit (320, 506) steuert, dass der Führungsdraht (100, 110, 210, 310, 510) einer Zielregion bereitstellt, um die Zielregion zu kühlen und innerhalb eines Zieltemperaturbereichs unterhalb normaler Körpertemperatur für eine verlängerte Zeitspanne hält.

## Revendications

1. Fil-guide (10, 110, 310, 510) à utiliser avec un dispositif thérapeutique, le fil-guide (10, 110, 310, 510) comprenant :
un élément allongé (25, 125) conformé pour son insertion dans un vaisseau corporel de telle façon qu'un dispositif thérapeutique peut être guidé par-dessus l'élément allongé (25, 125) et jusque dans le vaisseau corporel,
l'élément allongé (25, 125) ayant un lumen de perfusion (30, 130) s'étendant à travers lui-même depuis un orifice d'entrée de fluide (16, 520) jusqu'à des ouvertures de sortie incluses dans une portion distale de l'élément allongé (25, 125),
**caractérisé en ce que**
la portion distale de l'élément allongé (25, 125) inclut une pluralité de nervures (44, 144) s'étendant longitudinalement et un matériau d'engrènement expansible inclus entre chacune des nervures (44, 144),
chacune des nervures (44, 144) s'étendant sur une distance depuis le voisinage de l'extrémité distale de l'élément allongé (25, 125), dans lequel le matériau d'engrènement couvre les ouvertures de sortie, et dans lequel la construction de ladite portion distale présente un état rétracté qui facilite l'introduction de l'élément allongé (25, 125) dans le vaisseau corporel et à travers celui-ci, est un état en expansion dans lequel l'aire des ouvertures de sortie dans la portion distale de l'élément allongé (25, 125) est augmentée.

2. Fil-guide (10, 110, 210, 310, 510) selon la revendication 1, dans lequel le fil-guide (10, 110, 210, 310, 510) est utilisé avec un cathéter à ballon (50, 340) et présente une taille telle qu'un cathéter à ballon (50, 340) peut être guidé par-dessus l'élément allongé (25, 125, 225).

3. Fil-guide (10, 110, 210, 310, 510) selon la revendication 1 ou 2, dans lequel les nervures s'étendant longitudinalement s'étendent généralement en ligne droite dans l'état rétracté et de façon généralement cintrée vers l'extérieur dans l'état en expansion.

4. Fil-guide (10, 110, 210, 310, 510) selon l'une des revendications précédentes, dans lequel les nervures s'étendant longitudinalement comprennent des tiges à cintrer qui s'étendent généralement en ligne droite dans l'état rétracté et de façon généralement cintrée vers l'extérieur dans l'état en expansion.

5. Fil-guide (10, 110, 210, 310, 510) selon l'une des revendications précédentes, comprenant en outre un fil attaché à l'extrémité distale de l'élément allongé, de sorte qu'une traction sur la structure allongée en direction proximale amène une extrémité distale de la portion distale de l'élément allongé à être tirée plus proche d'une extrémité proximale de la portion distale de l'élément allongé, ce qui amène les nervures s'étendant longitudinalement à se cintrer vers l'extérieur.

6. Fil-guide (10, 110, 210, 310, 510) selon la revendication 5, dans lequel la structure allongée s'étend en direction proximale depuis un point d'attache au niveau de l'extrémité distale de l'élément allongé, à l'intérieur du lumen de perfusion (30, 130) de l'élément allongé, et hors du lumen de perfusion (30, 130) au voisinage de l'extrémité proximale de l'élément allongé.

7. Fil-guide (10, 110, 210, 310, 510) selon l'une des revendications précédentes, dans lequel les nervures s'étendant longitudinalement sont formées d'une composition comprenant un matériau à mémoire de forme.

8. Fil-guide (10, 110, 210, 310, 510) selon l'une des revendications précédentes, dans lequel le matériau d'engrènement expansible est formé d'une composition comprenant un matériau à mémoire de forme.

9. Fil-guide (10, 110, 210, 310, 510) selon la revendication 7 ou 8, dans lequel le matériau à mémoire de forme comprend du nitinol ou du polycyclooctane.

10. Fil-guide (10, 110, 210, 310, 510) selon l'une des revendications précédentes, dans lequel le diamètre extérieur du point central d'une section de perfusion située au niveau de la portion distale de l'élément allongé dans un état en expansion possède un diamètre qui est au moins deux fois le diamètre du même point quand la portion distale de l'élément allongé est dans un état rétracté.

11. Fil-guide (10, 110, 210, 310, 510) selon l'une des revendications précédentes, dans lequel une portion distale de l'élément allongé (25, 125, 225) est expansible lors de l'application d'un fluide refroidi (320, 506) à la portion distale de l'élément allongé (25, 125, 225) depuis le lumen de perfusion (30, 130).

12. Système pour exécuter une procédure avec cathéter à ballon, le système comprenant :
un fil-guide (10, 110, 210, 310, 510) selon l'une des revendications précédentes ;
un cathéter à ballon expansible (50, 340) ; et
un système de commande (500) qui commande la perfusion de fluide (320, 506) via le lumen de perfusion du fil-guide et hors des ouvertures de sortie dans la portion distale de l'élément allongé (25, 125, 225).

13. Système selon la revendication 12, comprenant en outre un système de commande (500) qui commande le gonflage et le dégonflage du ballon expansible (70).

14. Système selon les revendications 12 ou 13, dans lequel le système de commande (500) commande une quantité de fluide refroidi (320, 506) que le fil-guide (10, 110, 210, 310, 510) fournit à une région cible, de manière à refroidir et à maintenir la région cible dans une plage de température cible au-dessous de la température normale du corps pendant une période temporelle prolongée.
